# EUROPEAN PATENT APPLICATION

(11) **EP 3 978 123 A1**
(43) Date of publication of application: **06.04.2022**
(21) Application number: 21199545.1
(22) Date of filing: 28.09.2021
(51) Int. Cl.: B01L 3/00, B01D 57/02, B01L 7/00, C12Q 1/68, G01N 27/447

(54) **NUCLEIC ACID DETECTION KIT AND NUCLEIC ACID DETECTION DEVICE**

(30) Priority: 30.09.2020 US 202063085368 P; 30.09.2020 US 202063085385 P; 05.02.2021 US 202163146219 P; 01.07.2021 CN 202110746172
(71) Applicant: iCare Diagnostics International Co. Ltd., New Taipei 236 (TW); Century Technology (Shenzhen) Corporation Limited, Shenzhen, Guandong (CN)
(72) Inventor: CHANG, DENG-KAI, 236 New Taipei (TW); YANG, SU-CHIEN, 236 New Taipei (TW); TUNG, LI-YU, 236 New Taipei (TW); HSIEH, MING-YI, 236 New Taipei (TW); WU, HSIN-CHIEH, 236 New Taipei (TW); LU, TING-LAI, 236 New Taipei (TW); YANG, SHAO-FU, 236 New Taipei (TW); LIN, YUAN-TIEN, 236 New Taipei (TW); LEE, TAI-HSING, 236 New Taipei (TW)
(74) Representative: Zaboliene, Reda

(57) **Abstract**

A nucleic acid detection kit (100) includes a detection chip (2), an electrophoresis box (3), and a connection unit (8). The detection chip (2) includes interconnected channel (5), outlet (51), and first opening (29). The electrophoresis box (3) is disposed below the detection chip (2). The electrophoresis box (3) includes an inlet (37) corresponding to the channel (5) and a second opening (36) away from the channel (5). The outlet (51) is connected to the inlet (37). One end of the connection unit (8) is connected to the first opening (29), and the other end is connected to the second opening (36), so that the channel (5) is further connected to the electrophoresis box (3) for pressure equalization during transportation and sudden movements. A nucleic acid detection device (200) able to receive the nucleic acid detection kit (100) is also disclosed.

## Description

### FIELD

The subject matter relates to nucleic acid detection devices, and more particularly, to a nucleic acid detection kit and a nucleic acid detection device with the nucleic acid detection kit.

### BACKGROUND

Molecular diagnosis, morphological detection, and immunological detection are mostly carried out in laboratories. The detection process includes performing a polymerase chain reaction (PCR) amplification reaction in a large and medium-sized detection equipment to acquire an amplified product. Then, the amplified product is manually transferred to an electrophoresis detection equipment for an electrophoretic detection. Finally, an electrophoretic detection result is manually transferred to a fluorescence analyzer to obtain a fluorescence image. However, such detection process is time-consuming, inefficient, and inflexible, and the detection device is not portable. The detection cannot be carried out anytime and anywhere.

### SUMMARY

The present disclosure provides a nucleic acid detection kit, including a detection chip, an electrophoresis box, and a connection unit. The detection chip includes a first cover plate, a spacer layer, and a second cover plate, two opposite surfaces of the spacer layer are in contact with the first cover plate and the second cover plate, the first cover plate, the spacer layer, and the second cover plate cooperatively define a channel, the first cover plate defines an outlet, the second cover plate defines a first opening, each of the outlet and the first opening is connected to the channel, the channel is configured to cany a microbead, the microbead performs a PCR amplification reaction to obtain a mixed microbead in the channel, the electrophoresis box is disposed on a side of the first cover plate away from the channel, the electrophoresis box includes an inlet corresponds to the channel and a second opening away from the channel, the outlet is connected to the inlet, the mixed microbead enters the electrophoresis box through the outlet and the inlet, one end of the connection unit is connected to the first opening, and the other end of the connection unit is connected to the second opening, so that the channel is further connected to the electrophoresis box,

In some embodiments, the connection unit includes a first connection end connected to the first opening, a second connection end connected to the second opening, and a connection structure between the first connection end and the second connection end, and the first connection end is connected to the second connection end through the connection structure.

In some embodiments, the connection unit further includes a receiving cavity, the receiving cavity is disposed between the second connection end and the connection structure, and the second connection end is connected to the connection structure through the receiving cavity.

In some embodiments, the connection unit is a connection tube.

In some embodiments, the connection unit further includes a first sidewall, a second sidewall, a third sidewall, a bottom plate, and a top plate, the first sidewall is disposed on a surface of the second cover plate away form the channel, the second sidewall and the third sidewall are disposed on a surface of the electrophoresis box close to the detection chip, the third sidewall is disposed close to the spacer layer, the second sidewall is disposed away from the spacer layer, the bottom plate is disposed on a surface of the second cover plate and connected to the third sidewall, the top plate is disposed on one end of the second sidewall away from the electrophoresis box and extended to the first sidewall, the first sidewall, the second sidewall, the third sidewall, the bottom plate, the top plate, and the surface of the electrophoresis box close to the detection chip cooperatively define a hollow space, the first opening extends through the bottom plate and is connected to the hollow space, and the second opening is connected to the hollow space.
In some embodiments, the first opening further extends through the top plate.

In some embodiments, the electrophoresis box further includes a capillary, one end of the capillary extends through the inlet and the outlet to connect to the channel, and the other end of the capillary extends in the electrophoresis box,

In some embodiments, the electrophoresis box further includes an electrophoresis groove, a gel medium disposed in the electrophoresis groove, a liquid injection slot disposed on an end of the gel medium, the other end of the capillary away from the channel extends into the liquid injection slot.

In some embodiments, the first cover plate is disposed on an opening of the electrophoretic groove, the outlet and the inlet are a single through hole.

The present disclosure further provides a nucleic acid detection device, including the above nucleic acid detection kit and a host. A mounting groove is disposed on the host the nucleic acid detection kit is detachably disposed in the mounting groove.

The nucleic acid detection kit provided by the present disclosure integrates the PCR amplification reaction and the electrophoresis detection of nucleic acid into in a single piece of equipment. Thus, the nucleic acid detection kit has a simple structure, which is portable, flexible, and convenient, and can be used at home. The connection unit disposed between the detection chip and the electrophoresis box, prevents the silicone oil in the channel and the wetting liquid in the electrophoresis box from leaking out or being mixed together, and the reliability of the nucleic acid detection kit is improved.

### BRIEF DESCRIPTION OF THE DRAWINGS

Implementations of the present technology will now be described, by way of example only, with reference to the attached figures.
FIG. 1 is a front, top perspective view of an embodiment of a nucleic acid detection kit according to the present disclosure
FIG. 2 is a rear, bottom perspective view of an embodiment of a nucleic acid detection kit according to the present disclosure,
FIG. 3 is an exploded diagrammatic view of an embodiment of a nucleic acid detection kit according to the present disclosure.
FIG. 4 is an exploded diagrammatic view of an embodiment of a nucleic acid detection kit without a kit body according to the present disclosure.
FIG. 5 is a cross-sectional view of an embodiment of a detection chip of the kit according to the present disclosure,
FIG. 6 is a diagrammatic view of an embodiment of a driving circuit of a detection chip according to the present disclosure.
FIG. 7 is a diagrammatic view of an embodiment of an electrophoresis box according to the present disclosure.
FIG. 8 illustrates a microbead entering an electrophoresis box from a detection chip according to one embodiment of the present disclosure.
FIG. 9 illustrates a microbead entering an electrophoresis box from a detection chip according to another embodiment of the present disclosure.
FIG. 10 illustrates a microbead entering an electrophoresis box from a detection chip according to another embodiment of the present disclosure.
FIG. 11a is a cross-sectional view of an embodiment of a connection unit between a first opening and a second opening of a nucleic acid detection kit according to the present disclosure.
FIG. 11b is a cross-sectional view of another embodiment of a connection unit between a first opening and a second opening of a nucleic acid detection kit according to the present disclosure.
FIG. 12 is a diagrammatic view of an embodiment of a connection unit between a first opening and a second opening of a nucleic acid detection kit according to the present disclosure.
FIG. 13 is a cross-sectional view of an embodiment of a connection unit between a first opening and a second opening of a nucleic acid detection kit according to the present disclosure.
FIG. 14 is a diagrammatic view of an embodiment of a nucleic acid detection device according to the present disclosure.

### DETAILED DESCRIPTION

It will be appreciated that for simplicity and clarity of illustration, where appropriate, reference numerals have been repeated among the different figures to indicate corresponding or analogous components. In addition, numerous specific details are set forth in order to provide a thorough understanding of the embodiments described herein. However, it will be understood by those of ordinary skill in the art that the embodiments described herein can be practiced without these specific details. In other instances, methods, procedures, and components have not been described in detail so as not to obscure the related relevant feature being described. Also, the description is not to be considered as limiting the scope of the embodiments described herein. The drawings are not necessarily to scale, and the proportions of certain parts may be exaggerated to better illustrate details and features of the present disclosure.

The term "comprising," when utilized, means "including, but not necessarily limited to"; it specifically indicates open-ended inclusion or membership in the so-described combination, group, series, and the like.

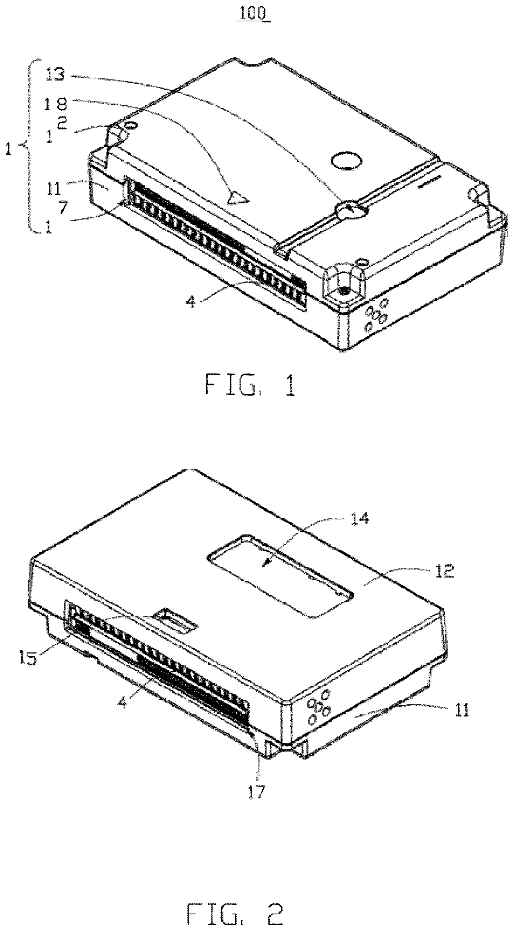

FIGS. 1, 3 to 5, illustrate a nucleic acid detection kit 100, which includes a kit body 1, a detection chip 2, and an electrophoresis box 3, and a connector 4. The detection chip 2 and the electrophoresis box 3 connected together are arranged in the kit body 1. The detection chip 2 and the electrophoresis box 3 are electrically connected to the connector 4. The detection chip 2 is configured to perform a PCR amplification reaction. The electrophoresis box 3 is configured to perform an electrophoresis detection. The detection chip 2 includes a first cover plate 21, a spacer layer 22, and a second cover plate 23. Two opposite surfaces of the spacer layer 22 are in contact with the first cover plate 21 and the second cover plate 23. The first cover plate 21, the spacer layer 22, and the second cover plate 23 cooperatively define a channel 5 for carrying a testable material in a droplet of solution ("solution") to be detected. The solution in the channel 5 is in a form of microbead "a". The microbead "a" may undergo the PCR amplification reaction to obtain a mixed microbead "b". The mixed microbead "b" enters the electrophoresis box 3 to undergo the electrophoresis detection. An image collection unit (not shown) acquires a fluorescent image within the electrophoresis box 3. The nucleic acid detection kit 100 integrates with the detection chip 2 and the electrophoresis box 3, which has a small size, and is suitable for a portable use. After the PCR amplification reaction is completed, the electrophoresis detection can be carried out automatically. The two processes are performed in a single equipment, and the sampling accuracy is precise. Thus, the detection process is efficient and flexible.

Referring to FIGS. 1 to 5, the kit body 1 includes a first housing 11, a second housing 12, a sampling port 13 disposed on the second housing 12, and a detection window 14 disposed on the first housing 11, The first housing 11 and the second housing 12 are connected together to define a receiving cavity (not shown in the figures). The detection chip 2, the electrophoresis box 3, and the connector 4 are disposed in the receiving cavity. The sampling port 13 is configured to correspond to the detection chip 2, through which the microbead "a" can be added. into the detection chip 2. The detection window 14 is configured to correspond to the electrophoresis box 3, so that the image collection unit can collect the fluorescent image of the electrophoresis box 3 through the detection window 14.

In an embodiment, the first housing 11 and the second housing 12 are assembled together by a latch or snapped together. The first housing 11 and the second housing 12 are further fastened together by screws to increase a connection strength therebetween.

In an embodiment, referring to FIGS. 1 to 3, a sidewall of the kit body 1 defines an opening 17. A connector 4, which is electrically connected to an external power supply (not shown), is installed in the opening 17. The connector 4 is disposed in the receiving cavity, and exposed through the opening 17 to facilitate the electrical connection between the connector 4 and the external power supply.

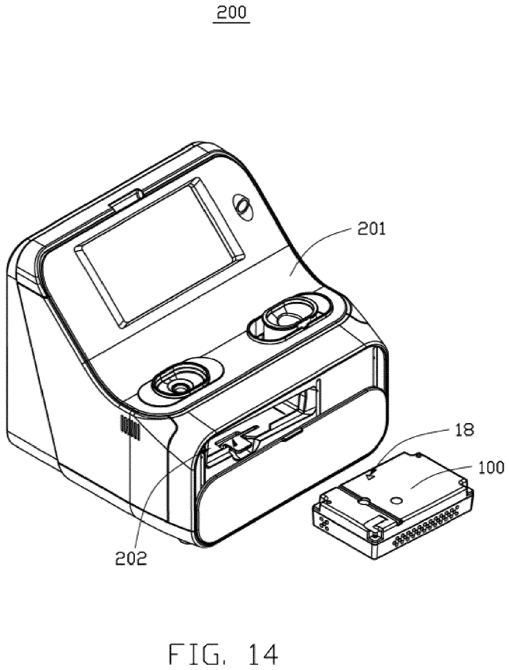

In an embodiment, referring to FIG. 2, the first housing 11 further defines a card slot 15. Referring to FIG. 14, a clamping structure (not shown) on a detection kit mounting groove 202 of a nucleic acid detection device 200 is configured to enter the card slot 15 to clamp the nucleic acid detection kit 100 in place.

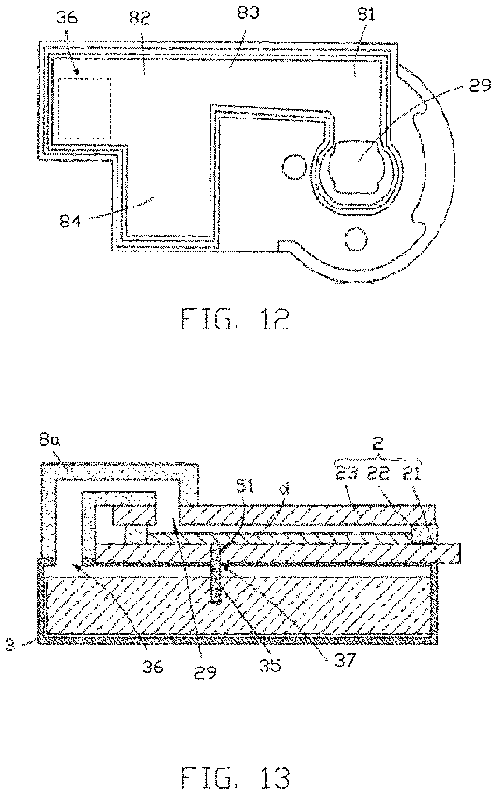

In an embodiment, referring to FIG. 1, an indication mark 18 (such as an arrow) is disposed on a side of the second housing 12 away from the receiving cavity. Referring to FIG. 13, the indication mark 18 is configured to indicate a direction of insertion of the nucleic acid detection kit 100 into the nucleic acid detection device 200, to avoid wrong insertion.

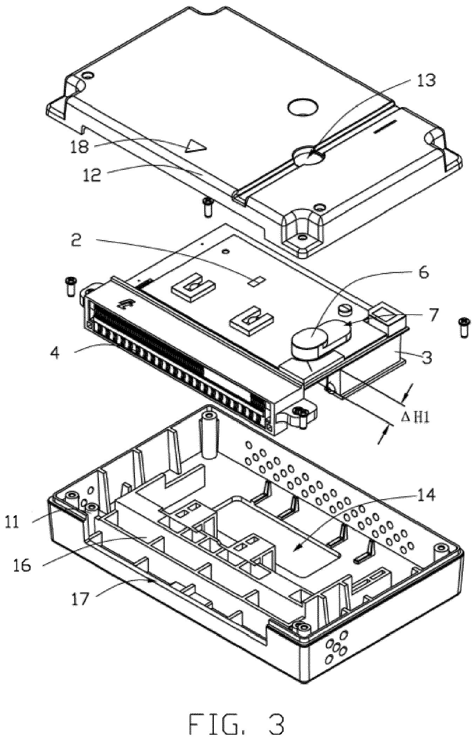

In an embodiment, referring to FIG. 3, several support structures 16 are disposed in the kit body 1. Due to thicknesses of the detection chip 2, the electrophoresis box 3, and the connector 4 being different, support structures 16 of different heights are disposed to support the detection chip 2, the electrophoresis box 3, and the connector 4 in the kit body 1, achieving connection stability of the nucleic acid detection kit 100.

In an embodiment, the kit body 1 may be made of, but is not limited to, plastic.

In an embodiment, the support structures 16, the first housing 11, and the second housing 12 are an integrated structure.

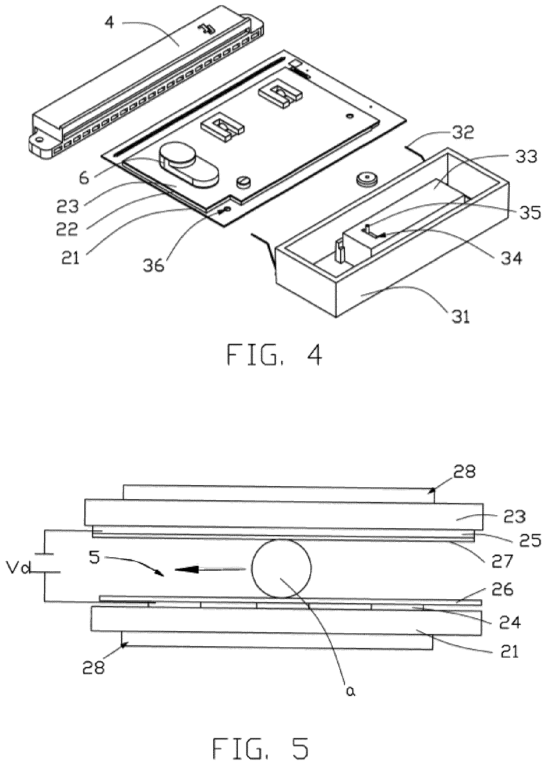

Referring to FIGS. 4 and 5, the detection chip 2 further includes a driving circuit 24, a first dielectric layer 26, a conductive layer 25, and a second dielectric layer 27. The driving circuit 24 is disposed on a surface of the first cover plate 21 close to the second cover plate 23. The first dielectric layer 26 is disposed on a side of the driving circuit 24 close to the second cover plate 23. The conductive layer 25 is disposed on a surface of the second cover plate 23 close to the first cover plate 21. The second dielectric layer 27 is disposed on a side of the conductive layer 25 close to the first cover plate 21. The driving circuit 24 and the conductive layer 25 are electrically connected to the connector 4. The microbead "a" can be moved along a flow path in the channel 5 by energizing or de-energizing circuits between the driving circuit 24 and the conductive layer 25.

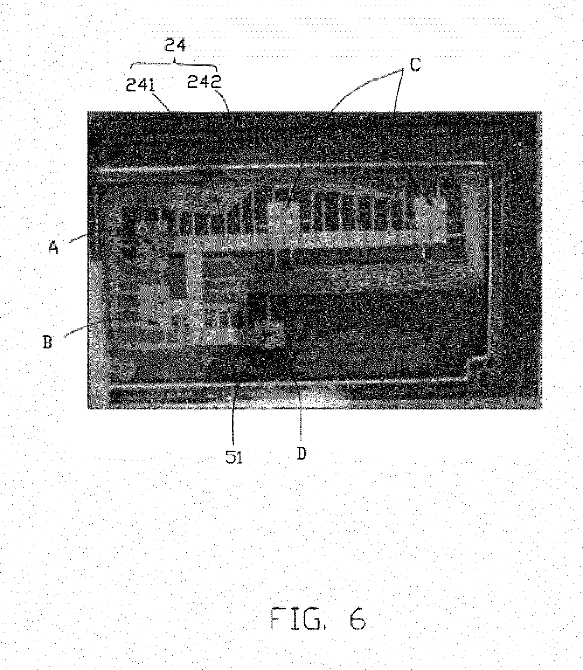

Referring to FIGS. 1, 5 and 6, the driving circuit 24 includes a plurality of driving electrodes 241 disposed in an array and a plurality of control electrodes 242. Each of the driving electrodes 241 is electrically connected to one of the control electrodes 242. The control electrodes 242 are further electrically connected to the connector 4. In an embodiment, the driving circuit 24 is a thin film transistor (TFT) driving circuit. The microbead "a" has some conductivity, and can be driven by circuits applying wetting and de-wetting voltages to dielectric (EWOD) between the driving electrodes 241 and the conductive layer 25 to move along the flow path in the channel 5. In an EWOD operation, one of the circuits between one of the driving electrodes 241 and the conductive layer 25 can be selectively energized to change wetting characteristics between the microbead "a" and the first dielectric layer 26 and between the microbead "a" and the second dielectric layer 27, so as to control the microbead "a" to move along the flow path. Referring to FIG. 6, the driving electrodes 241 include a driving electrode "I", a driving electrode "H". and a driving electrode "G". The microbead "a" moves on the driving electrode "I", the driving electrode "H", and the driving electrode "G". When the microbead "a" is on the driving electrode "H", a voltage "Vd" is applied between the driving electrode "G" and the conductive layer 25, and the driving electrode "H" is disconnected from the conductive layer 25. At this time, the wetting characteristics between the microbead "a" and the first dielectric layer 26, and between the microbead "a" and the second dielectric layer 27 are changed, so that a liquid-solid contact angle between the driving electrode "H" and microbead "a" becomes larger, and a liquid-solid contact angle between the driving electrode "G" and microbead "a" becomes smaller, to promote the movement of the microbead "a" from the driving electrode "H" to the driving electrode "G".

In an embodiment, the first dielectric layer 26 and the second dielectric layer 27 are insulated and are hydrophobic. On the one hand, the first dielectric layer 26 and the second dielectric layer 27 have the characteristics of insulation and hydrophobicity, and on the other hand, the first dielectric layer 26 and the second dielectric layer 27 can make the microbead "a" move smoothly along the flow path to avoid breakage or fragmentation of the microbead "a" during movement.

In an embodiment, each of the first dielectric layer 26 and the second dielectric layer 27 may be, but is not limited to, a polytetrafluoroethylene coating.

Referring to FIG. 5, in an embodiment, the driving circuit 24 may be formed on the surface of the first cover plate 21 by metal etching or electroplating.

In an embodiment, the control electrodes 242 are integrated at an edge of the first cover plate 21. An electrical connection between the detection chip 2 and the connector 4 is realized by inserting the side of the first cover plate 21 with the control electrodes 242 into the connector 4.

Referring to FIGS. 3, 6 and 8, in an embodiment, the driving circuit 24 can be divided into a plurality of areas according to their different purposes, including a sample adding area "A", a reagent storage area "B". a plurality of PCR amplification areas "C", and an outlet area "D". The detection chip 2 corresponding to the sample adding area "A" defines a first opening 29. The first opening 29 is configured to correspond to the sampling port 13 on the second cover plate 23. The microbead "a" is added in the sampling area "A" through the sampling port 13 and the first opening 29. The reagent storage area "B" is configured to store fluorescent reagents (such as fluorescent dyes or fluorescent probes). The microbead "a" undergoes the PCR amplification reaction in the PCR amplification areas "C" to form an amplified product first. The amplified product is then mixed with a fluorescent reagent in the reagent storage area "B" to form the mixed microbead "b". The outlet area "D" is connected to the electrophoresis box 3. The outlet area "D" includes an outlet 51, The mixed microbead "b" enters the electrophoresis box 3 through the outlet 51. The number of the PCR amplification areas "C" can be determined according to an actual detection requirement.

Referring to FIGS. 5 and 6, after the microbead "a" enters the sampling area "A", the microbead "a" moves to the PCR amplification areas "C" and undergoes the PCR amplification reaction to form an amplified product. When the PCR amplification reaction is completed, the amplified product is moved to the reagent storage area "B" and mixed with the fluorescent reagent to obtain the mixed microbead "b". The mixed microbead "b" then enters the electrophoresis box 3 through the outlet 51 and undergoes the electrophoretic detection.

In order to mix the amplified product and the fluorescent reagent more evenly, the mixed microbead "b" is moved back and forth several times in the PCR amplification area "C. A mixing area (not shown) can also be set in the driving circuit 24 to mix the amplified product and the fluorescent reagent therein.

In an embodiment, the number of the PCR amplification areas "C" is two. The temperatures of the two PCR amplification areas C for heating purposes are different, which realizes different stages of the PCR amplification reaction.

In an embodiment, the fluorescent reagent (such as a fluorescent dye or a DNA probe) is within the reagent storage area "B" in advance. Thus, there is no need to add fluorescent reagent in the detection chip 2 separately.

In yet another embodiment, referring to FIG. 3, the fluorescent reagent can also be added in the detection chip 2 to mix with the amplified product. The detection chip 2 defines a reagent tank 7 corresponding to the reagent storage area "B", and the fluorescent reagent can be added into the reagent tank 7 during the PCR amplification reaction. The type of the fluorescent reagent can be selected according to an actual need, which can improve the flexibility of the PCR amplification reaction.

Referring to FIGS. 3, 5, and 6, the detection chip 2 further includes a heating unit 28. The heating unit 28 is disposed on a surface of the first cover plate 21 away from the channel 5 and / or on a surface of the second cover plate 23 away from the channel 5. The heating unit 28 corresponds to the PCR amplification areas "C" and is connected to the connector 4. The heating unit 28 heats the microbead "a" to undergo the PCR amplification reaction.

In an embodiment, two heating units 28 are disposed on a surface of the first cover plate 21 away from the channel 5 and on a surface of the second cover plate 23 away from the channel 5.

In an embodiment, the heating unit 28 is disposed on the surface of the second cover plate 23 away from the channel 5 through a thermally conductive adhesive layer (not shown).

Referring to FIG. 3 and 5, in an embodiment, the first cover plate 21 and the second cover plate 23 are glass plates. The spacer layer 22 is a frame with adhesive on both sides, which is connected to edges of the first cover plate 21 and the second cover plate 23 to cooperatively define the channel 5. A volume of the channel 5 can be adjusted by changing a thickness of the spacer layer 22 according to an actual demand.

In an embodiment, silicone oil "d" may be injected into the channel 5 after the detection chip 2 is assembled, and the microbead "a" is driven to move in the silicone oil.

Referring to FIG. 3, 4, 7, 8, and 11a, the electrophoresis box 3 includes an electrophoresis body 31, two electrophoretic electrodes 32, a gel medium 33, and a capillary 35. Two electrophoretic electrodes 32 are disposed. on two ends of the electrophoresis body 31. The electrophoretic body 31 defines an electrophoretic groove 314. The gel medium 33 is disposed in the electrophoretic groove 314. A liquid injection slot 34 is disposed on an end of the gel medium 33. Each of the two electrophoresis electrodes 32 is electrically connected to the connector 4. The electrophoresis box 3 further includes an inlet 37. The inlet 37 corresponds to the outlet 51. The capillary 35 includes a first end 351 and a second end 352. The first end 351 extends into the channel 5 through the inlet 37 and the outlet 51. The second end 352 extends into the liquid injection slot 34. The capillary 35 is filled with wetting liquid. The mixed microbead "b" on the outlet area "D" which is in contact with the wetting liquid in the capillary 35 may enter into the liquid injection slot 34 of the gel medium 33 through the outlet 51, the inlet 37, and the capillary 35, thereby undergoing the electrophoresis detection in the electrophoresis box 3.

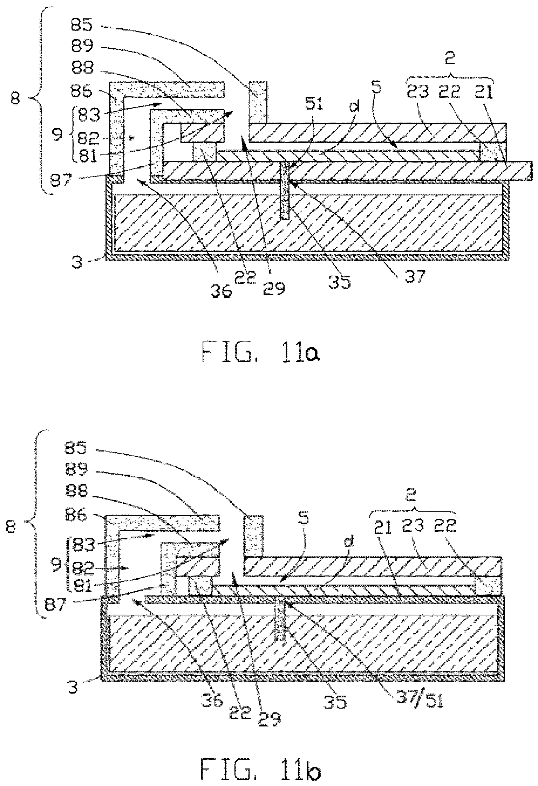

Referring to FIGS. 3, 4, 7, and 8, the electrophoresis body 31 is disposed on a side of the first cover plate 21 away from the second cover plate 23. An opening of the electrophoresis body 31 faces the first cover plate 21. The electrophoresis body 31 includes a transparent substrate 311 and a plurality of sidewalls 312 connected to the transparent substrate 311. The transparent substrate 311 and the sidewalls 312 cooperatively define the electrophoretic groove 314. Ends of the sidewalls 312 away from the transparent substrate 311 are connected to a surface of the first cover plate 21 away from the channel 5, thereby, the electrophoresis body 31 is covered by the first cover plate 21. Referring to FIG. 11b, since the first cover plate 21 is the cover plate of the electrophoretic groove 314, the outlet 51 and the inlet 37 are actually one single orifice. With the above configuration, a height difference "ΔH₁" is defined between the silicone oil "d" in the detection chip 2 and the wetting liquid in the electrophoresis box 3. The height difference "ΔH₁" enables the mixed microbead "b" in the channel 5 to enter the electrophoresis box 3 smoothly. The electrophoresis box 3 can better connect to the detection chip 2, which facilitates the transfer of the mixed microbead "b" from the detection chip 2 to the electrophoresis box 3. The nucleic acid detection kit 100 integrates with the detection chip 2 and the electrophoresis box 3, which has a small size.

In an embodiment, a sealing rubber ring (not shown) is disposed between the sidewall 312 and the first cover plate 21 to improve the sealing performance of the electrophoresis box 3.

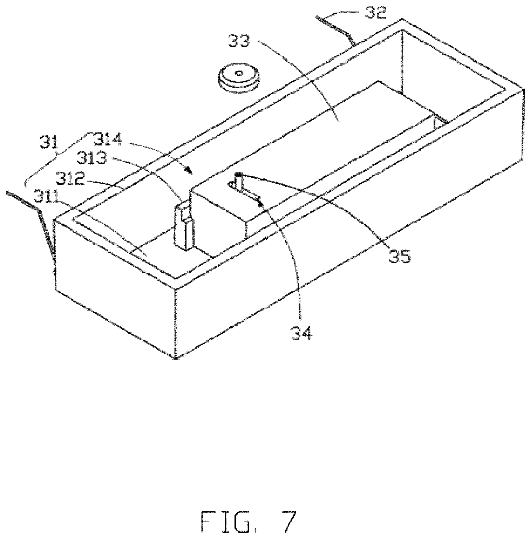

Referring to FIG. 7, the electrophoresis body 31 further includes a plurality of clamping portions 313 disposed on the transparent substrate 311. The clamping portions 313 can fix the gel medium 33 in place, thereby guaranteeing the accuracy of the electrophoresis detection.

In an embodiment, the gel medium 33 is substantially cubic.

In an embodiment, referring to FIG. 3, the transparent substrate 311 is a transparent glass plate, and the fluorescence image of the electrophoresis box 3 can be observed on a side of the transparent substrate 311 away from the gel medium 33.

In an embodiment, the number of the clamping portions 313 is four. Four clamping portions 313 are disposed outside of the gel medium 33 to fix the gel medium 33.

Referring to FIG. 4, the electrophoresis body 31 further includes a second opening 36 disposed on the first cover plate 21. The second opening 36 corresponds to the electrophoresis box 3.

In an embodiment, a buffer can be injected into the electrophoresis body 31 through the second opening 36.

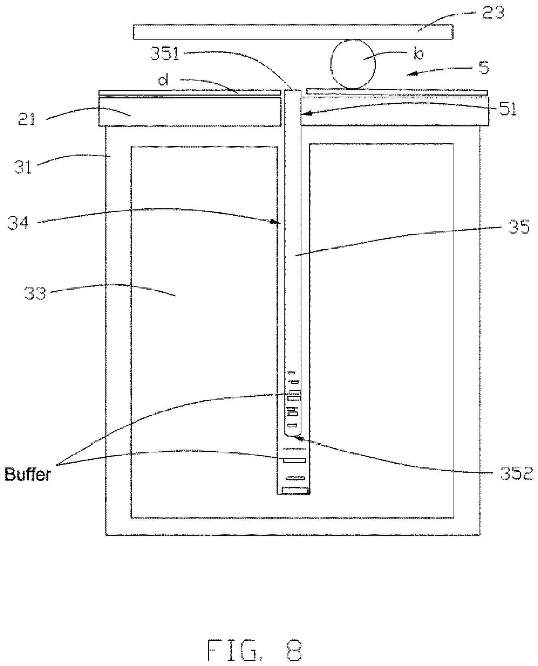

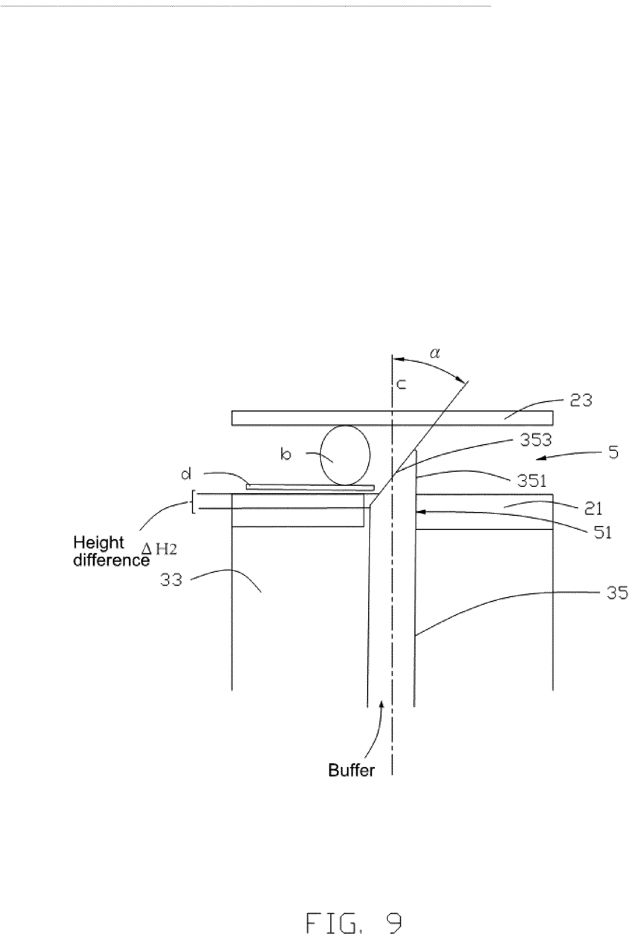

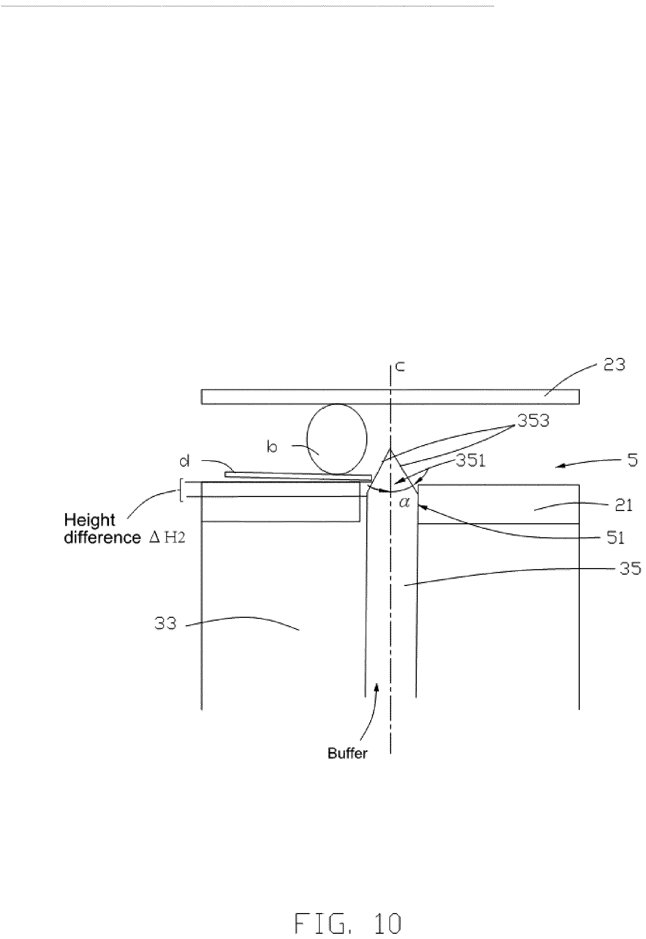

Referring to FIGS. 4, and 8 to 10, the first end 351 of the capillary 35 extends into the channel 5 through the outlet 51, The outlet 51 is disposed on the first cover plate 21. Due to a capillary effect, the mixed microbead "b" enters the gel medium 33 through the capillary 35. Referring to FIG. 8, to facilitate the mixed microbead "b" entering the electrophoresis box 3 smoothly, the first end 351 needs to be flush with the liquid level of the silicone oil "d". In another embodiment, referring to FIGS. 9 and 10, the first end 351 includes at least one inclined plane 353, The lowest point of the inclined plane 353 is lower than the bottom surface of the channel 5. There is a height difference "ΔH₂" between the lowest point of the inclined plane 353 and the bottom surface of the channel 5. A liquid level of the silicone oil "d" is equal to the inclined plane 353, which allows the mixed microbead "b" to smoothly enter the capillary 35. During the assembly of the capillary 35 and the detection chip 2, the capillary 35 needs to be filled with buffer, and the buffer in the capillary 35 needs to be in contact with the mixed microbead "b" in the outlet area "D" to form a continuous liquid flow. Then, the mixed microbead "b" can enter the electrophoresis box 3 smoothly under the capillary effect.

In an embodiment, an angle between the inclined plane 353 and a central axis "C" of the capillary 35 ranges from 45 degrees to 60 degrees. Then, the inclined plane 353 functions to enable the mixed microbead "b" to enter the electrophoresis box 3 smoothly.

In an embodiment, referring to FIG. 9, liquid inlet 351 incudes one inclined plane 353. An angle between the inclined plane 353 and the central axis "C" of the capillary 35 ranges from 45 degrees to 60 degrees.

In yet another embodiment, referring to FIG. 10, liquid inlet 351 includes two inclined planes 353. An angle between each of the inclined planes 353 and the central axis "C" of the capillary 35 ranges from 45 degrees to 60 degrees.

Referring to FIG. 4, one end of each electrophoresis electrode 32 extends into the electrophoresis body 31, and the other end is electrically connected to the connector 4.

Referring to FIGS. 3 and 8, due to the height difference "ΔH₁" between the silicone oil "d" in the detection chip 2 and the wetting liquid in the electrophoresis box 3, the wetting liquid will not enter the channel 5 through the capillary 35 under normal conditions. Since the surface of the first end 351 of the capillary 35 is flush with the surface of the silicone oil "d", the silicone oil "d" in the channel 5 will not enter the electrophoresis box 3 through the capillary 35. However, the nucleic acid detection kit 100 may be tilted or be vibrated during transportation, and internal pressures of the detection chip 2 and the electrophoresis box 3 may be changed during the transportation. The silicone oil "d" in the channel 5 and the wetting liquid in the electrophoresis box 3 may leak out or be mixed together, which may affect the performance of the nucleic acid detection kit 100, and the nucleic acid detection kit 100 may lose its function.

Referring to FIGS. 1 and 11a, the nucleic acid detection kit 100 further includes a connection unit 8. The connection unit 8 is disposed between the detection chip 2 and the electrophoresis box 3, which avoids the leakage and the mixing of the silicone oil "d" and the wetting liquid. . One end of the connection unit 8 is connected to the first opening 29 of the detection chip 2, and the other end is connected to the second opening 36 of the electrophoresis box 3, so another connection is formed between the channel 5 and the electrophoresis box 3. Thus, the pressures in the channel 5 and the electrophoresis box 3 are always balanced, avoiding leakage and the mixing of the silicone oil "d" and the wetting liquid.

Referring to FIG, 11a, the connection unit 8 includes a first connection end 81 connected to the first opening 29, a second connection end 82 connected to the second opening 36, and a connection structure 83 between the first connection end 81 and the second connection end 82. The first connection end 81 is connected to the second connection end 82 through the connection structure 83, When the pressure in the channel 5 is greater than the pressure in the electrophoresis. box 3, a part of the silicone oil "d" may be forced into the connection structure 83 through the first opening 29. When the pressure in the electrophoresis box 3 is greater than the pressure in the channel 5, a part of the wetting liquid may be forced into the connecting structure 83 through the second opening 36, so as to balance the pressures in the channel 5 and the electrophoresis box 3. Thus, the leakage and the mixing of the silicone oil "d" in the channel 5 and the wetting liquid in the electrophoresis box 3 is avoided.

In an embodiment, referring to FIGS. 11a and 12, the connection unit 8 further includes a receiving cavity 84. The receiving cavity 84 is disposed between the second connection end 82 and the connection structure 83. The second connection end 82 is connected to the connection structure 83 through the receiving cavity 84. The receiving cavity 84 is configured to receive any silicone oil "d" forced from the channel 5 and wetting liquid forced from the electrophoresis box 3. Thus, leaking and mixing of the silicone oil "d" in the channel 5 and the wetting liquid in the electrophoresis box 3 is restrained.

Referring to FIG. 11a, the electrophoresis box 3 is disposed below the detection chip 2. and the electrophoresis box 3 is arranged not aligned with the detection chip 2. Along a direction perpendicular to an extension direction of the vertical channel 5, a projection area of the electrophoresis box 3 is greater than a projection area of the detection chip 2. The connection unit 8 further includes a first sidewall 85, a second sidewall 86, a third sidewall 87, a bottom plate 88, and a top plate 89. The first sidewall 85 is disposed on a surface of the second cover plate 23 away from the channel 5. The second sidewall 86 and the third sidewall 87 are disposed on a surface of the electrophoresis. box 3 close to the detection chip 2. The third sidewall 87 is disposed close to the spacer layer 22. The second sidewall 86 is disposed away from the spacer layer 22. The bottom plate 88 is disposed on a surface of the second cover plate 23 and connected to the third sidewall 87. The top plate 89 is disposed on one end of the second sidewall 86 away from the electrophoresis box 3 and is extended to the first sidewall 85. The first sidewall 85, the second sidewall 86, the third sidewall 87, the bottom plate 88, the top plate 89, and the surface of the electrophoresis box 3 close to the detection chip 2 cooperatively define a hollow space 9. The hollow space 9 includes the first connection end 81, the second connection end 82, the connection structure 83, and the receiving cavity 84. The first opening 29 extends through the bottom plate 88 and is connected to the hollow space 9. The second opening 36 is connected to the hollow space 9.

In yet another embodiment, referring to FIG. 11b, the first cover plate 21 covers the opening of the electrophoresis groove 314. The outlet 51 and the inlet 37 are actually a single through hole. An area of the first cover plate 21 is larger than an area of the second cover plate 23. A region of the first cover plate 21 extends out of the second cover plate 23 and forms a bottom of the connection unit 8. The first sidewall 85, the second sidewall 86, the third sidewall 87, the bottom plate 88, the top plate 89, and the surface of the first cover plate 21 away from the electrophoresis body 31 cooperatively define the hollow space 9. The assembly of the first cover plate 21 as a cover plate of the electrophoresis body 31 and the electrophoresis box 3 is facilitated, and a volume of the nucleic acid detection kit 100 can be reduced.

In an embodiment, referring to FIG. 3 and 11a, the first opening 29 further extends through the top plate 89 and connects with the sampling port 13, so that a nucleic acid sample can be added into the sampling area "A".

In yet another embodiment, referring to FIG. 13, the connection unit 8a may be a connection tube. One end of the connection unit 8a is connected to the first opening 29, and the other end of the connection unit 8a is connected to the second opening 36, so as to balance the pressures in the channel 5 and the electrophoresis box 3. Moreover, the connection unit 8a is simple and easy to assemble. The connection unit 8a may be made of rubber, which can flex to occupy the smallest space in the nucleic acid detection kit 100.

FIG. 14 illustrates a nucleic acid detection device 200 according to the present disclosure. The nucleic acid detection device 200 includes a host 201, and the nucleic acid detection kit 100, A mounting groove 202 is disposed on the host 201. The nucleic acid detection kit 100 is detachably disposed in the mounting groove 202.

With the above configuration, the nucleic acid detection kit 100 provided by the present disclosure integrates the PCR amplification reaction and the electrophoresis detection of nucleic acid into in a single piece of equipment. Thus, the nucleic acid detection kit 100 has a simple structure, which is portable, flexible, and convenient, and can be used at home. The connection unit disposed between the detection chip 2 and the electrophoresis box 3, prevents the silicone oil "d" in the channel 5 and the wetting liquid in the electrophoresis box 3 from leaking out or being mixed together, and the reliability of the nucleic acid detection kit 100 is improved.

The embodiments shown and described above are only examples. Even though numerous characteristics and advantages of the present technology have been set forth in the foregoing description, together with details of the structure and function of the present disclosure, the disclosure is illustrative only, and changes may be made in the detail, including in matters of shape, size and arrangement of the parts within the principles of the present disclosure, up to and including, the full extent established by the broad general meaning of the terms used in the claims.

## Claims

1. A nucleic acid detection kit (100), **characterized in that**, comprising:
a detection chip (2);
an electrophoresis box (3); and
a connection unit (8);
the detection chip (2) comprises a first cover plate (21), a spacer layer (22), and a second cover plate (23), two opposite surfaces of the spacer layer (22) are in contact with the first cover plate (21) and the second cover plate (23), the first cover plate (21), the spacer layer (22), and the second cover plate (23) cooperatively define a channel (5), the first cover plate (21) defines an outlet (51), the second cover plate (23) defines a first opening (29), each of the outlet and the first opening (29) is connected to the channel (5), the channel (5) is configured to carry a microbead (a), the microbead (a) performs a PCR amplification reaction to obtain a mixed microbead (b)in the channel (5);
the electrophoresis box (3) is disposed on a side of the first cover plate (21) away from the channel (5), the electrophoresis box (3) comprises an inlet (37) corresponds to the channel (5) and a second opening (36) away from the channel (5), the outlet (51) is connected to the inlet (37), the mixed microbead (b) enters the electrophoresis box (3) through the outlet (51) and the inlet (37), one end of the connection unit (8) is connected to the first opening (29), and the other end of the connection unit (8) is connected to the second opening (36), so that the channel (5) is further connected to the electrophoresis box (3).

2. The nucleic acid detection kit (100) of claim 1, **characterized in that**, the connection unit (8) comprises a first connection end (81) connected to the first opening (29), a second connection end (82) connected to the second opening (36), and a connection structure (83) between the first connection end (81) and the second connection end (82).

3. The nucleic acid detection kit (100) of claim 2, **characterized in that**, the connection unit (8) further comprises a receiving cavity (84), the receiving cavity (84) is disposed between the second connection end (82) and the connection structure (83), and the second connection end (82) is connected to the connection structure (83) through the receiving cavity (84).

4. The nucleic acid detection kit (100) of claim 1, **characterized in that**, the connection unit (8) is a connection tube.

5. The nucleic acid detection kit (100) of claim 1, **characterized in that**, the connection unit (8) further comprises a first sidewall (85), a second sidewall (86), a third sidewall (87), a bottom plate (88), and a top plate (89), the first sidewall (85) is disposed on a surface of the second cover plate (23) away from the channel (5), the second sidewall (86) and the third sidewall (87) are disposed on a surface of the electrophoresis box (3) close to the detection chip (2), the third sidewall (87) is disposed close to the spacer layer (22), the second sidewall (86) is disposed away from the spacer layer (22), the bottom plate (88) is disposed on a surface of the second cover plate (23) and connected to the third sidewall (87), the top plate (89) is disposed on one end of the second sidewall (86) away from the electrophoresis box (3) and extended to the first sidewall (85), the first sidewall (85), the second sidewall (86), the third sidewall (87), the bottom plate (88), the top plate (89), and the surface of the electrophoresis box (3) close to the detection chip (2) cooperatively define a hollow space (9), the first opening (29) extends through the bottom plate (88) and is connected to the hollow space (9), and the second opening (36) is connected to the hollow space (9).

6. The nucleic acid detection kit (100) of claim 5, **characterized in that**, the first opening (29) further extends through the top plate (89).

7. The nucleic acid detection kit (100) of claim 1, **characterized in that**, the electrophoresis. box (3) further comprises a capillary (35), one end of the capillary (35) extends through the inlet (37) and the outlet (51) to connect to the channel (5), and the other end of the capillary (35) extends in the electrophoresis box (3).

8. The nucleic acid detection kit (100) of claim 7, **characterized in that**, the electrophoresis box (3) further comprises an electrophoresis groove (314), a gel medium (33) disposed in the electrophoresis groove (314), a liquid injection slot (34) disposed on an end of the gel medium (33), the other end of the capillary (35) away from the channel (5) extends into the liquid injection slot (34).

9. The nucleic acid detection kit (100) of claim 8, **characterized in that**, the first cover plate (21) is disposed on an opening of the electrophoretic groove (314), the outlet (51) and the inlet (37) are a single through hole.

10. A nucleic acid detection device (200), **characterized in that**, comprising:
a nucleic acid detection kit (100) of any one of claims 1 to 9;
a host (201), a mounting groove (202) is disposed on the host (201), the nucleic acid detection kit (100) is detachably disposed in the mounting groove (202).
